# EUROPEAN PATENT SPECIFICATION

(11) **EP 0 696 596 B1**
(45) Date of publication and mention of the grant of the patent: **30.08.2000**
(21) Application number: 95302900.6
(22) Date of filing: 28.04.1995
(51) Int. Cl.: C07K 16/16, C07K 16/02, G01N 33/94, C07K 1/22, C07D 305/00

(54) **Detection, assay and isolation of compounds having the taxane ring skeleton**
Nachweis, Test und Gewinnung von Zusammensetzungen die ein Taxanringskelett enthalten
Détection, titrage et isolation de composés à squelette de noyau de taxane

(30) Priority: 19.07.1994 IT MI941499
(43) Date of publication of application: 14.02.1996
(73) Proprietor: INDENA S.p.A., 20139 Milano (IT)
(72) Inventor: Bombardelli, Ezio, I-20141 Milano (IT); Concetti, Antonio, I-63100 Ascoli Piceno (IT); Venanzi, Franco M., I-62032 Camerino MC (IT)
(74) Representative: Ritter, Stephen David

(56) References cited:
- WO-A-94/20134
- BIOLOGICAL CHEMISTRY HOPPE-SEYLER, vol. 375, no. 6, June 1994 BERLIN, GERMANY, pages 419-423, A. CONCETTI ET AL. 'Immunorecognition of ring skeleton of taxanes by chicken egg yolk antibodies.'
- JOURNAL OF FOOD SCIENCE, vol. 58, no. 4, July 1993 CHICAGO, IL, USA, pages 739-742, T. HORIKOSHI ET AL. 'IgG antibody from hen egg yolks: Purification by ethanol fractionation.'
- JOURNAL OF IMMUNOLOGICAL METHODS, vol. 158, no. 1, 14 January 1993 AMSTERDAM, THE NETHERLANDS, pages 5-15, P. GROTHAUS ET AL. 'An enzyme immunoassay for the determination of taxol and taxanes in Taxus sp. tissues and human plasma.'
- BIOLOGICAL CHEMISTRY HOPPE-SEYLER, vol. 375, no. 4, April 1994 BERLIN, GERMANY, pages 281-287, Y. GUO ET AL. 'Immunological detection and quantitation of 10-deacetylbaccatin III in Taxus sp. plant and tissue cultures.'

## Description

This invention relates to the detection, assay and isolation of compounds having the taxane ring skeleton, in particular taxol and 10-deacetylbaccatin III.

### Background of the invention

Taxol is a complex diterpene pseudoalkaloid which can be extracted from the bark of the western yew *Taxus brevifolia* and has been reported to display anti-cancer activity in otherwise refractory solid tumors (Chabner, B.A. Principles and Practice of Oncology 5 (1991) 1). In the face of an increasing demand for Taxol, the availability of the drug is unfortunately limited because of the scarcity of the substance (its concentration in the bark is approx 0.01%), and the difficulties met during its purification. Current supplies of *Taxus sp.* are insufficient to provide sufficient drug to treat patient populations such as those with metastatic ovarian and breast cancers.

As a consequence, the possibility of using Taxol in medicine is dependent upon methods being developed for obtaining it from renewable sources. (Appendino, G., Fitoterapia 1 (vol. XIV suppl.) (1993) 5). At the moment the only practical alternative to bark harvesting is the hemisynthesis of Taxol. One procedure is based on the isolation from yew needles of 10-deacetylbaccatin III (DAB), a compound that can be converted into Taxol, from which it differs only in not having an phenylisoserine chain in position C-13 and an acetyl group in position C-10. The content of DAB in leaves of different *Taxus* species is very variable but generally much higher (up to 10 times) than the Taxol content in the bark of the Pacific yew (Fitoterapia, *supra*).

Taxol and 10-deacetylbaccatin III have the structural formulae (1) and (2) respectively as shown below:

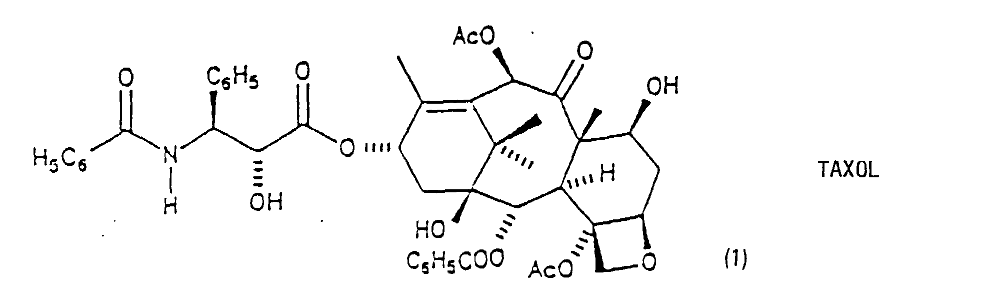

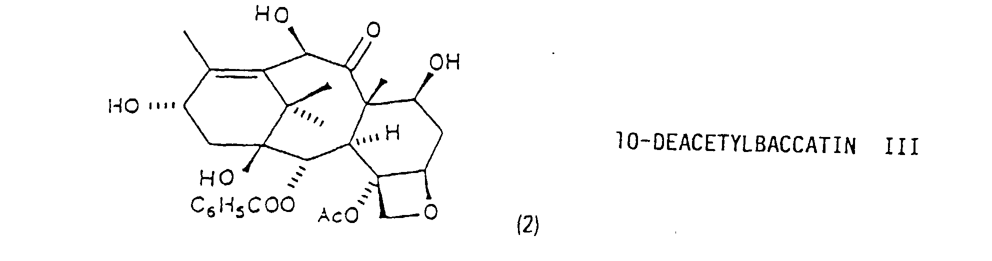

These compounds, their derivatives and related compounds, known collectively as taxanes, are substances with antitumour activity that are obtainable from plants of the genus Taxus.

Several procedures for converting DAB into Taxol have been reported (see e.g. Holton, R.A., Eur. Pat. Appl. EP 0 400,971 (1990): Chem. Abstr. 114, 164568q (1990) and Ojima, I. Habus, I. Zhao, M., Zucco, M., Hook Park, Y., Ming Sun, C., Brigaud, T., Tetrahedron 48 (1992) 6985). Further, DAB can be modified by esterification of the hydroxyl group in position C-13 with various amino acid derivatives. An approach of this type have led to the production of so-called "Taxoters", i.e. hemisynthetic analogues of Taxol, which also displays a strong antitumor activity (Gueritte-Voelgelein, F. Guenard, D. Lavelle, F. Le Goff, M-T. L. Potier, P.J. Med Chem 34 (1991) 992). The hemisynthetic strategy would be significantly improved if it was possible to select varieties of yew with a high content of valuable Taxol precursors (i.e. 10-deacetylbaccatin III, and baccatinIII). However in view of the complex chemical structure of taxol and its potential precursors, chemical and physico-chemical assay methods tend to be inefficient.

The isolation of taxanes in their pure form from vegetable sources involves complex chromatographic separations with final yields that are not very high.

Immunological procedures have the potential to speed up and simplify the laborious chromatographic analysis of taxanes which are present as a complex mixture in crude plant extracts (Fitoterapia, *supra*), but although polyclonal (pAb) and monoclonal antibodies (mAb), suitable for quantification of taxol-containing biological samples, have already been developed (Jaziri, M., Diallo, B.M., Vanhalelen, M.H., Vanhalelan-Fastre, R.J., Zhiri, A., Becu, A.C., Homes, J.J. Pharm. Belg. 46 (1991) 93, (Grothaus, P.G., Raybould, T.J.G., Bignami, G.S.; Lazo, C.B.; Byrnes, J.B. J. Immunol Methods 158 (1993) 5 and (Leu, J-C., Chen, B-X., Schiff, P.B., Erlanger, B.F., Cancer Research, 53 (1993) 1388, none effectively satisfies the need for an efficient assay for Taxol precursors.

Also, immunological processes, when they can be applied on an industrial scale to extract substances present in very small quantities in plants or cell or microorganism culture media, and particularly in complex and heterogeneous mixtures, have the advantage of accelerating and simplifying laborious chromatographic separations.

The preparation of antitaxol and antibaccatin antibodies isolated from mammals is reported in the literature (Yanwen Guo et al., Biol. Chem. Hoppe-Seyler, 375, 281; 1994). However, these antibodies are expensive to produce so there is a need to find an alternative, less expensive source of antibodies. The provision of an alternative and superior source of suitably specific antibodies would additionally satisfy the need for binding reagents useful in procedures for isolating Taxol and its precursors.

It has now been found that avian IgY antibodies, e.g. chicken antibodies from egg yolk are able to interact specifically with the ring system of compounds having the taxane ring skeleton, e.g. DAB-like taxanes. These antibodies have utility as reagents for the search and selection of new sources of Taxol precursors as alternatives to Pacific or European yews, in analyticac methods and also in the development of procedures for isolating such compounds.

### Summary of the invention

Thus, it has now been found that by immunizing avian females, e.g. hens with suitable immunogenic conjugates of taxol and 10-deacetylbaccatin III, polyclonal antibodies specific to these substances can be produced simply, without the need to take blood from any animal, and in virtually unlimited quantities. The production of these antibodies and their accumulation in the egg yolk in eggs of avian females that have been immunized in fact continues for over a year, bringing considerable advantages in terms of industrial production costs. The ease of isolation from the biological material is a further advantage in industrial terms since these antibodies, in the handling stages and when fixed to the matrix, withstand the use of relatively high concentrations of organic solvents, which means that the medium can be kept sterile for long periods without the addition of preservatives that could affect the activity of the antibody. This property also means that the crude antibodies can be stored for long periods and safely collected before under-going final purification and/or being used for various applications.

The quantity of antigens necessary for the immunization is far smaller than (often no more than a tenth of) that usually used for the production of antibodies in mammals.

The antibodies prepared according to the invention have the ability to interact specifically with compounds which have, for example, the carbon skeleton of 10-deacetylbaccatin III. It is therefore possible to distinguish (by immunizing avian females with the specific derivatives) between 10-deacetylbaccatin III and taxol. Compounds that can advantageously be isolated by this technology include 10-deacetylbaccatin III, baccatin III and 14-hydroxy-10-deacetylbaccatin III.

The same technology can be used to prepare other specific antibodies to taxanes, such as cephalomannin and 7-epitaxol, which normally constitute impurities of the main products of interest. The present invention therefore has two main objects, namely the analytical determination by an immunodiagnostic method of the above compounds and their quantitative isolation from various substrates.

The antibodies that are the subject of the present invention, which are produced by avian females, e.g. hens have surprisingly proved to have advantageous stability characteristics by comparison with similar products obtained from mammals, particularly when used on aqueous and non-aqueous vegetable extracts, since they are resistant to the common organic solvents used during the preparation and extraction processes; these antibodies also have the advantage that they can be used in mixtures of water with organic solvents such as ethanol and alcohols miscible with water acetone up to concentrations that permit complete solubilization of the antigen.

Thus according to one aspect of the present invention there is provided an antibody of the avian IgY class which has antibody specificity for an antigen which comprises the taxane ring skeleton.

The invention further provides a method of producing an antibody as defined above which comprises challenging an avian female with said antigen and isolating the IgY antibody from the subsequently laid eggs.

The taxane ring skeleton (carbon atoms only being shown and without stereochemical designations) is based upon the following C-20 tricyclic structure

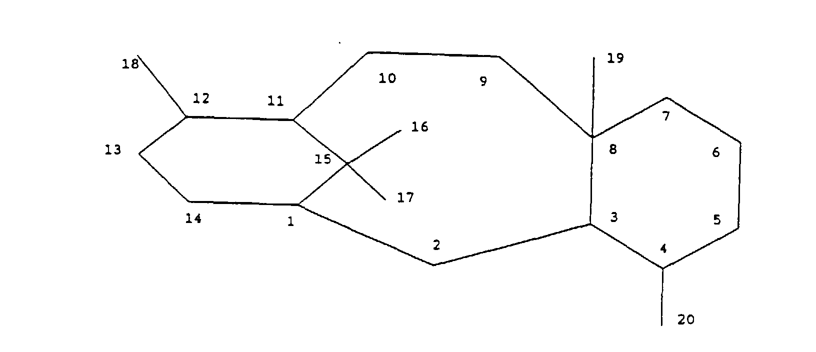

Exemplary compounds have the following formulae

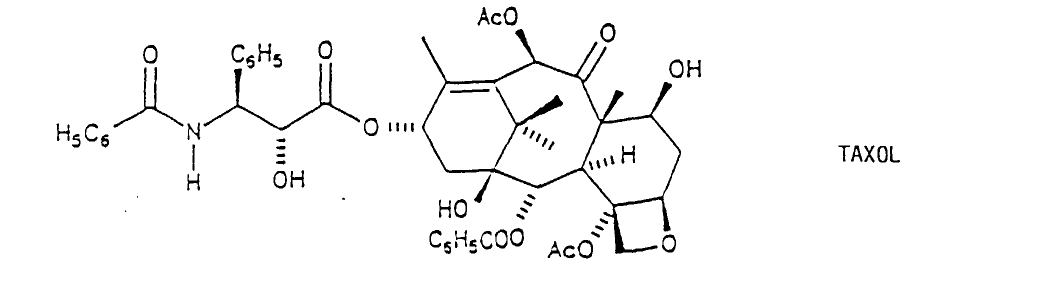

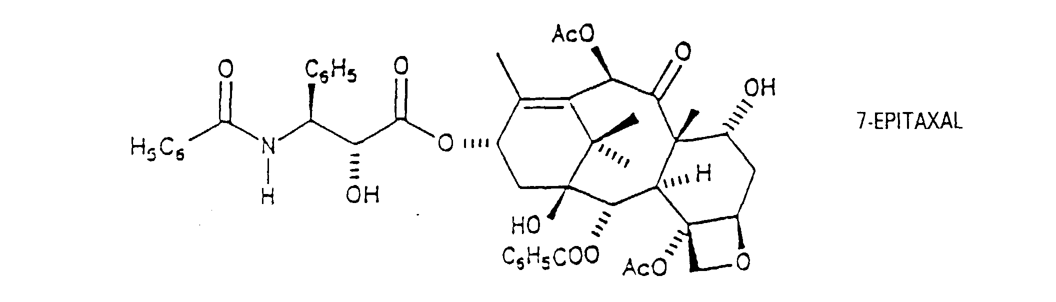

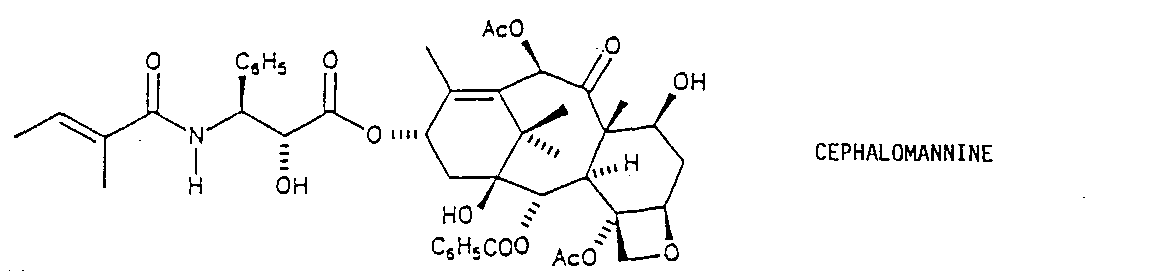

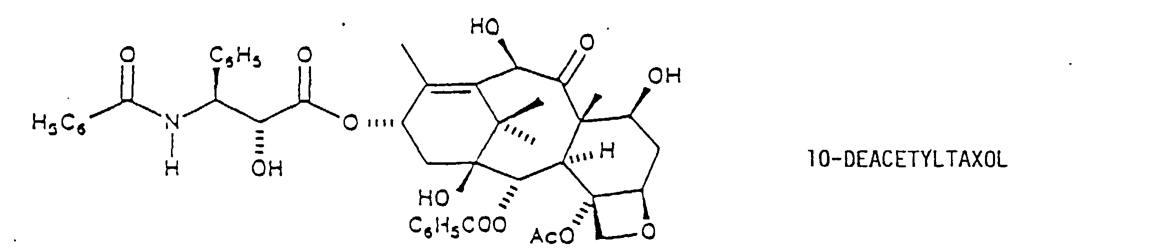

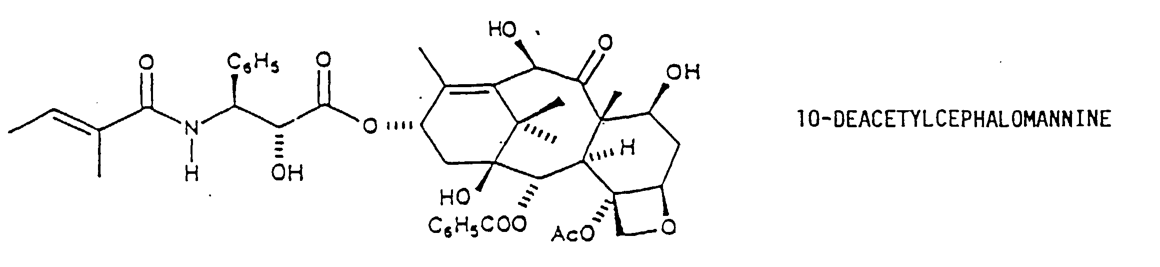

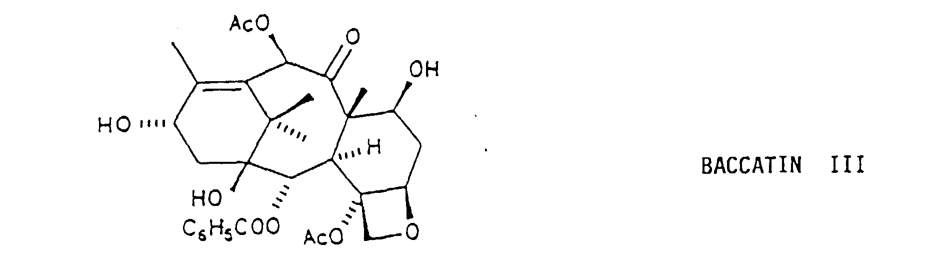

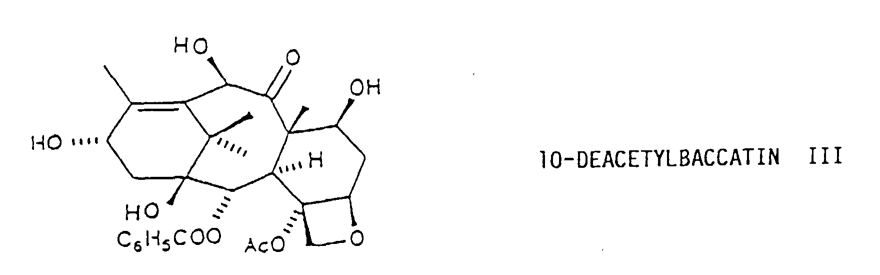

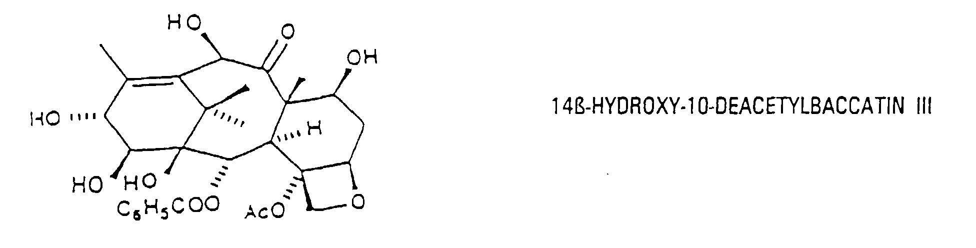

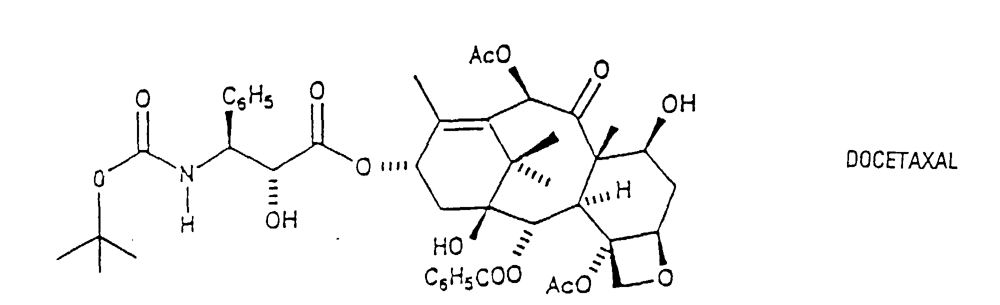

IgY antibodies produced according to the invention are useful as analytical reagents for detecting and/or assaying a compound comprising the taxane ring skeleton and as a preparation reagent for use in isolating or purifying a compound comprising the taxane ring skeleton. In particular, in view of their selectivity for the diterpene rings of DAB-like taxanes, the IgYs of the invention can be used for the immunological screening of a large number of plants which should enable new sources of Taxol precursors to be discovered.

Therefore a further aspect of the present invention is a process for the purification of a taxane, comprising contacting a mixture containing the said taxane with a polyclonal antibody to the said taxane, subsequent isolation of the taxane-antibody complex and recovery of the taxane, characterized in that the said polyclonal antibody is an IgY antibody, especially one obtained from hens' eggs.

The polyclonal antibodies themselves and the process used for their preparation come within the scope of the present invention, as does the use of the abovementioned process.

The invention further provides a process which can be used on an analytical scale to assay (i.e. to detect or determine the presence of) taxanes in mixtures or biological fluids. Hence another aspect of the present invention is a method for the determination of taxanes in mixtures and biological fluids, characterized in that this method uses a polyclonal antibody as described above.

In particular, another aspect of the invention a method is provided for the determination of taxanes in samples of biological fluids or culture media, in particular blood, which involves treating the said sample with a polyclonal antibody as mentioned above and subsequently determining the taxane by an immunodiagnostic method.

The preparation of IgY antibodies according to the invention will now be described in more detail.

### Detailed description of the invention

In practical terms, a suitable derivative of taxane, such as taxol, 10-deacetylbaccatin III or one of their analogues, is prepared by reacting this compound with succinic anhydride in pyridine in the presence of 4-dimethylaminopyridine and isolating the succinyl-taxane chromatographically on a silica gel column or by equivalent methods. The succinyl-taxane thus obtained may be conjugated by processes known in the literature (B.F. Erlanger, Methods in Enzymol. 70, Van Vunakis (eds) Academic Press, 1980) by dissolving it in DMSO which is added to a solution of N-(3-dimethylaminopropyl)-N-ethylcarbodiimide hydrochloride in physiological saline. The derivative formed may reacted, again in physiological saline, with bovine serum albumin for 24 hours and then dialysed against distilled water.

These conjugates are administered to selected avian females, e.g. laying hens, with a total of 0.5 mg of conjugate of 10-deacetylbaccatin III or taxol and/or other taxanes with bovine serum albumin being injected subcutaneously into various parts of the body using Freund's adjuvant; boosters are given at brief intervals during the first month using 0.25 mg of conjugate. The egg yolks are separated from the whites and ruptured in phosphate buffer pH 7.5 containing 0.1M NaCI in accordance with procedures known in the literature (Polson et al., Immunol. Comm., 9, 475, 1980). After vigorous stirring, PEG (polyethylene glycol) is added to the suspension in solid form until the concentration is 3%, then the whole is centrifuged; the precipitate is discarded and further PEG is added to the supernatant until the concentration is 12%; the precipitate is collected by centrifuging and redissolved in Tris buffer pH 7.9 containing EDTA. The antibodies are purified using columns of activated Sepharose to which the serum albumin and the taxane derivative bind when the components are left to react in the presence of N-(3-dimethylaminopropyl)-N-ethylcarbodiimide hydrochloride.

The unreacted groups of the resin are saturated with acetic acid. The resin thus prepared is washed repeatedly with a 0.1M acetate buffer pH 4.5, 0.1M bicarbonate pH 8.3 containing 0.5M NaCI. The resin is now ready for purification of the antibodies which, once absorbed, can be re-eluted by washing the column with a 0.2M solution of glycine hydrochloride at pH 2.5.

Immunoaffinity chromatography is now a well-known technique for purifying numerous antigens. The technique uses the interaction between an immunoglobulin and a specific antigen, which in the case in question is a taxane, such as taxol or 10-deacetylbaccatin III or one of their analogues. Therefore, though this does not limit the scope of the invention, resins with a derivatized methacrylic structure, such as Affi-prep Hz hydrazide (Biorad) or resin columns again containing hydrazine groups such as the Avidchroms, are used as a support matrix to immobilize the antibodies. These resins have the ability to form stable links such as hydrazones with the oxidized carbohydrates of the immunoglobulins. The immunoglobulins are particularly rich in oxidizable carbohydrates compared with antibodies from other sources.

The antibodies immobilized on the matrix after they have fixed the relative antigen are treated by the normal procedures described in the literature to recover the antigen and regenerate the column. A solution of aqueous dioxane or aqueous aliphatic alcohols containing a buffer at pH 2.5, for example glycine/HCl, is normally used to recover the antigen. Other techniques, normally recommended by the suppliers of matrices for affinity chromatography, can also be used.

As far as the analytical aspect is concerned, the methods described above can be used to determine quantities of antigen in vegetable extracts, in culture media and in plasma after the administration of drugs (in a preferred case taxol) containing the products cited above in quantities of between 1 and 100 ng.

In another embodiment, the method according to the present invention can be carried out by another immunodiagnostic method, for example competitive inhibition enzyme immunoassay (CIEIA).

As far as the aspect of industrial preparation is concerned, the taxanes that are the subject of the invention can be directly "captured" by aqueous alcoholic extracts obtained by extracting, for example, vegetable biomasses with ethanol or methanol diluted by up to 50%, preferably around 40%, with water. This solvent has the advantage of extracting all the taxanes and not extracting unwanted substances such as chlorophyll and other pigments which are present in particularly high concentrations in the aerial parts of plants of the genus Taxus.

These extracts, without the need for further purification processes but merely after simple filtration, can be chromatographed through specific immunoaffinity columns. Because of the high resistance of the antibodies obtained according to the present invention to solvents and to the pH operating conditions, these columns can be used for at least 100 consecutive cycles. Taxol and 10-deacetylbaccatin III as well as other taxanes of oncological interest can be prepared by this method, with the desired antigens being produced by analogous procedures.

The method is in this sense also particularly useful for eliminating taxane impurities that are difficult to isolate by normal chromatographic procedures such as silica gel columns or high pressure reverse phase industrial columns; by virtue of its high specificity, immunoaffinity chromatography enables, for example, taxol to be separated from its isomers such as 7-epi-taxol or cephalomannin; it can also be used to isolate 10-deacetylbaccatin III and 14-hydroxybaccatin.

Naturally the method can be used to obtain the compounds mentioned in the pure state from botanical species in which they are present in extremely small amounts.

Species of the genus Taxus commonly used industrially are Taxus baccata, T. brevifolia, T. wallichiana, T hicksi, T. canadensis, T. hiamanensis, T. piramidalis as well as selected cultivars.

We give below various examples that illustrate the invention in greater detail.

### Example I -

### Preparation of an activated matrix with antitaxol polyclonal antibodies useful in isolation of taxol from extracts of Taxus baccata.

### Preparation of the antigen:

0.9 mmol taxol and benzyl alcohol are reacted with 1.4 mmol succinic anhydride in pyridine in the presence of 0.067 mmol 4-dimethylaminopyridine. The whole is kept at ambient temperature for 7 hours. The reaction mixture is concentrated to dryness under vacuum at low temperature and the succinyl taxol is isolated by chromatography on silica gel using as eluent an 85:15 mixture of CHCl₃ and methanol (yield 70%). According to the spectroscopic data, the derivative is 2'-succinyl-taxol.

### Conjugation of the antigen with bovine serum albumin:

30 mg 2'-succinyl taxol are added with stirring to a solution of 1-ethyl-3-(dimethylaminopropionyl)carbodiimide in 0.9% NaCI. The mixture is left to react at ambient temperature for 24 hours and then reacted with 120 mg bovine serum albumin in physiological saline for a further 24 hours. The reaction mixture is dialysed for a further 36 hours against distilled water to eliminate the reagents.

### Preparation of the substrate to purify the antibodies:

50 mg bovine serum albumin are reacted with Sepharose activated with CNBr and then the whole is reacted with 2'-succinyl-taxol as prepared above in the presence of 100 mg N'-(3-dimethylaminopropyl)-N-ethylcarbodiimide hydrochloride at pH 5-6 for 12 hours.

The excess of unreacted groups on the resin is saturated with acetic acid; the resin is then washed with 0.1M acetate buffer pH 4.5 with 0.1M NaHCO₃ containing 0.5M NaCl. When the resin is used for immunoaffinity separation it is washed with 0.2M glycine/HCl buffer pH 2.5.

### Immunization of the hens and preparation of the antibodies:

10 laying hens are injected subcutaneously in several places with a total of 0.5 mg each of taxol conjugate with bovine serum albumin using Freund's adjuvant; the procedure is repeated after 15 and 21 days. Egg collection starts from day 30. 60 eggs are broken and the yolks separated from the whites, which are discarded. The yolks are homogenized in 2 volumes of 0.01M phosphate buffer pH 7.5 containing 0.1M NaCl. With vigorous stirring, PEG 6000 is added to give a concentration of 3% (w/v); the suspension is then centrifuged for 20 minutes at 13,000 g and the precipitate discarded. With vigorous stirring PEG 6000 is added to the supernatant to give a concentration of 12%. The suspension is centrifuged and the solid containing the antibodies collected. The precipitate is redissolved in 50 mM Tris buffer containing EDTA and NaN₃ as stabilizer. The antibodies are purified by passing this solution over the resin described above containing the antigen. The antibodies are re-eluted at the time of use with 0.2M glycine/HCl buffer pH 2.5.

### Immobilization of the antitaxol polyclonal antibody on the matrix:

An Avid-chrom column with hydrazide groups equilibrated with 0.05M sodium acetate pH 5 is treated with a solution of antibodies that have been pre-oxidized with sodium periodate by leaving the matrix to react with the antibody solution for approximately 1 hour (30 mg antibodies purified as described above are dissolved in 30 ml 0.05M sodium acetate and oxidized with 10 mM sodium periodate). The column is washed at the end with 0.02M phosphate buffer pH 7.4 to remove the excess of unreacted antibodies.

### Isolation of taxol from an extract of Taxus baccata:

100 g of finely chopped Taxus baccata leaves are extracted with 35% aqueous ethanol to exhaustion in taxol (approx. 2 l solvent). After filtration this extract is passed over a 20 ml Avid-chrom column prepared as described above. This column fixed 12.6 mg taxol, which was the quantity previously determined in the leaves by HPLC. The taxol is removed from the column by washing it with a 1:1 mixture of dioxane and water containing glycine/HCl buffer pH 2.5. The taxol is extracted from the column eluate with methylene chloride and crystallized from alcohol. The purity of the taxol obtained is over 99%.

### Example II - Preparation of an activated matrix containing anti-10-deacetylbaccatin III polyclonal antibodies useful in the isolation of 10-deacetylbaccatin III from extracts of Taxus baccata.

The specific antibodies and the immunoaffinity column are prepared by the same scheme and methods as described in example I, but using a different antigen, which is prepared as follows:

### Preparation of the 10-succinyl-10-deacetylbaccatin III:

0.9 mmol 10-deacetylbaccatin III are reacted with 1.4 mmol succinic anhydride in pyridine in the presence of 0.067 mmol 4-dimethylaminopyridine. The whole is kept at ambient temperature for 7 hours. The reaction mixture is concentrated to dryness under vacuum at low temperature and the succinyl derivative of the 10-deacetylbaccatin III is isolated by chromatography on silica gel using as eluent a 7:3 mixture of CHCl₃/isopropanol. According to the spectroscopic data, the derivative is 10-succinyl-baccatin III.

### Isolation of 10-deacetylbaccatin III from extracts of Taxus wallichiana:

100 g of finely chopped Taxus wallichiana leaves are extracted with 45% aqueous ethanol to exhaustion in taxanes (approximately 2.8 I solvent). After concentration to two thirds of the initial volume and filtration, this extract is passed through a 60 ml Avid-chrom column prepared as described for taxol. This column fixed 30.5 mg 10-deacetylbaccatin III, which represents 95% of the quantity previously determined in the leaves by HPLC. The 10-deacetylbaccatin III is removed from the column by washing it with a 2:1 mixture of dioxane/water containing glycine/HCl buffer pH 2.5. The diterpene is extracted from the column eluate, after it had been diluted with water, with methylene chloride and crystallized from methanol.

The 10-deacetylbaccatin III thus obtained has a purity of over 99%.

### Example III - Determination of the 10-deacetylbaccatin III content of human serum by Competitive Inhibition Enzyme Immunoassay (CIEIA)

30 ng of the 10-deacetylbaccatin III-BSA conjugate are made to adhere to the wells of a microtitration plate. For the CIEIA tests, serial dilutions of the 10-deacetylbaccatin III antigen (from 0.1 ng/ml to 10 µg/ml) are mixed with its antibody, α-10-deacetylbaccatin III IgY (2.3 µg/ml) both in PBS-T and in serum diluted 1:50 with the same buffer. After preincubation, 100 µl of these solutions are incubated for 1 hour at 37°C in the wells for the competition with the adherent antigen. Under these conditions the quantity of competitor antigen that gives an IC50 of 0.7 ng in PBS-T is the same in the presence of serum diluted 1:50.

## Claims

1. An antibody of the avian IgY class which has antibody specificity for an antigen which comprises the taxane ring skeleton.

2. An antibody according to claim 1 wherein the antigen has one of the following structural formulae

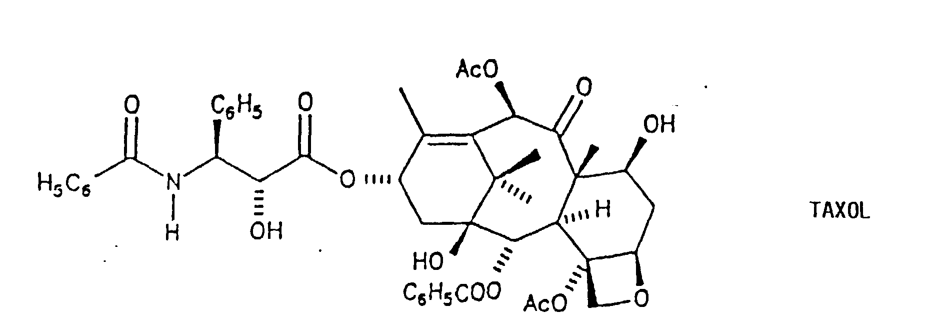

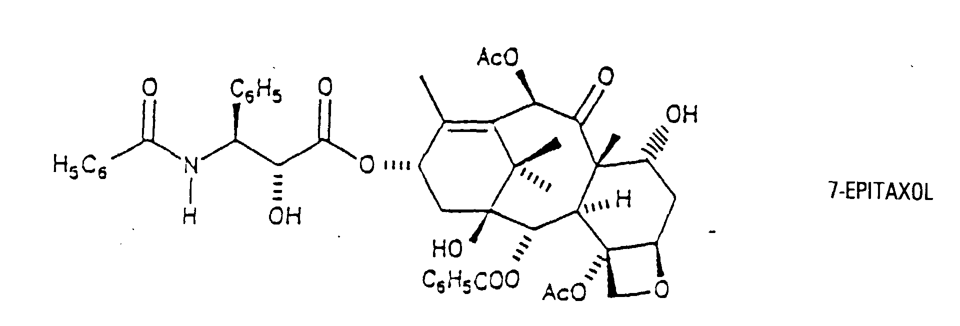

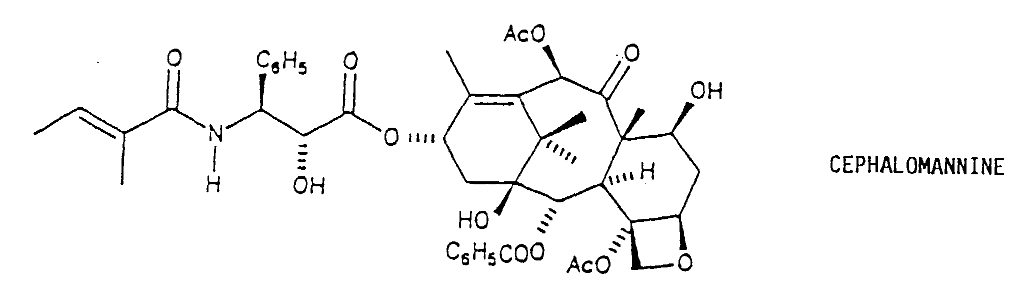

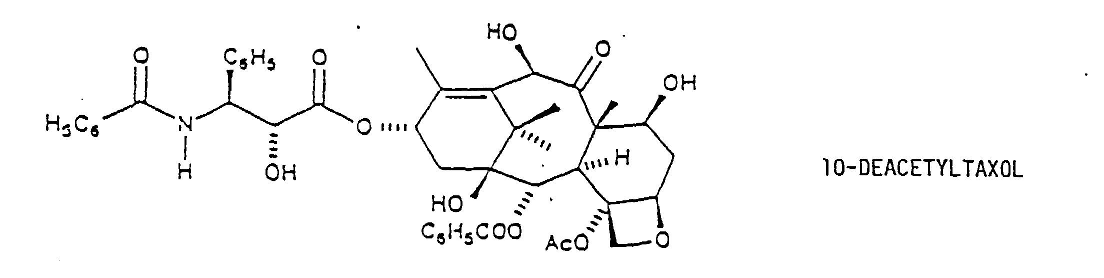

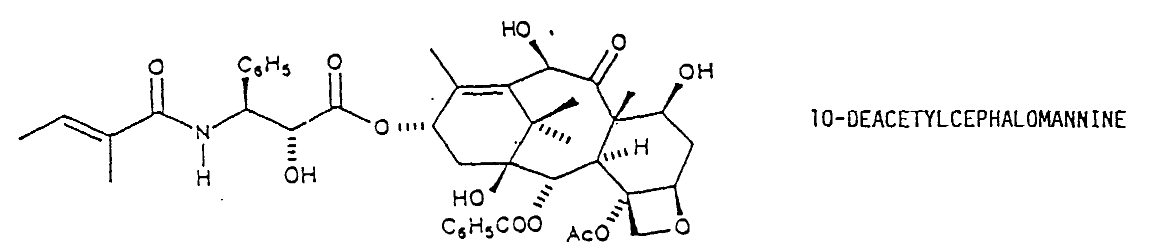

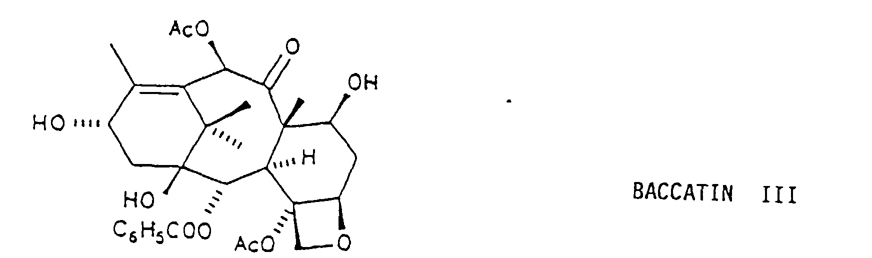

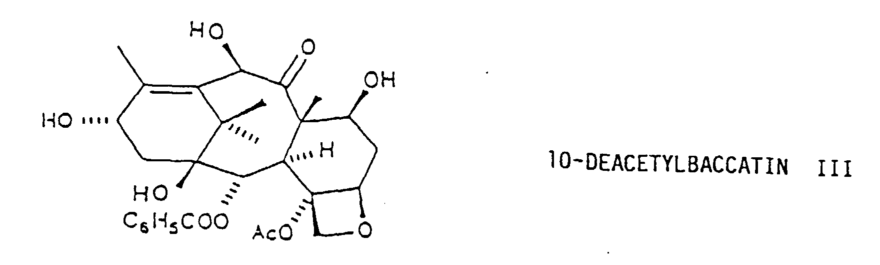

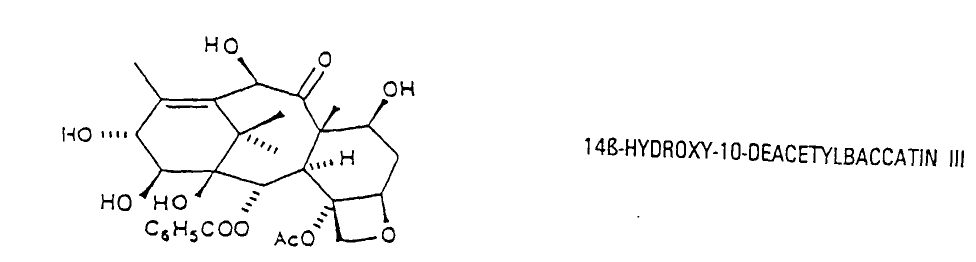

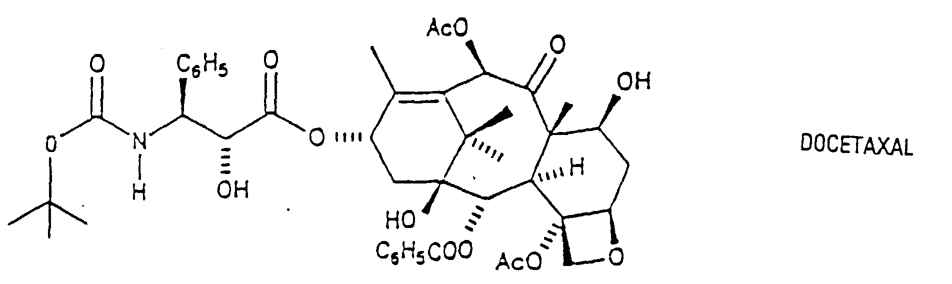

3. An antibody according to Claim 1 or Claim 2 wherein the antigen is taxol or a taxol precursor.

4. An antibody according to claim 3 wherein the antigen is 10-deacetyl baccatin III

5. An antibody according to any preceding claim which is a domestic hen IgY.

6. A method of producing an antibody as defined in any preceding claim which comprises challenging an avian female with said antigen and isolating the IgY antibody from the subsequently laid eggs.

7. The use of an antibody as claimed in any of Claims 1 to 5 as an analytical reagent for detecting and/or assaying a compound comprising the taxane ring skeleton.

8. The use of an antibody as claimed in any of Claims 1 to 5 as a preparative reagent for use in isolating or purifying a compound comprising the taxane ring skeleton.

9. Process for the purification of a taxane comprising the treatment of a mixture containing the said taxane with a polyclonal antibody to the said taxane, subsequent isolation of the taxane-antibody complex and recovery of the taxane, characterized in that the said polyclonal antibody is obtained from an avian species, especially from hens.

10. Process according to Claim 9, characterized in that the said polyclonal antibody is fixed on a chromatographic support for immunoaffinity chromatography.

11. Process according to Claim 9, characterized in that the said chromatographic support is a resin with a derivatized methacrylic structure with hydrazine groups.

12. Process according to Claim 11, characterized in that the elution of the said support is effected with an aqueous mixture of dioxane or an aliphatic alcohol buffered to pH 2.5.

13. Process according to Claim 12, characterized in that the eluent is a mixture of dioxane and water buffered with glycine/HCl.

14. Process according to Claim 9, characterized in that the said mixture is an extract obtained from a species of the genus Taxus chosen from the group consisting of Taxus baccata, T. brevifolia, T. wallichiana, T. hicksi, T. canadensis, T. hiamanensis, T. piramidalis.

15. Process according to any of Claims 9 to 14, characterized in that the taxane isolated is selected from the group consisting of taxol, 10-deacetylbaccatin III, 14-hydroxy-10-deacetylbaccatin III, baccatin III, cephalomannine, 19-hydroxy-10-deacetylbaccatin III and 7-epitaxol.

16. Process according to any of Claims 9 to 14, characterized in that the taxane isolated is taxol and the eluent is a 1:1 mixture of dioxane and water buffered to pH 2.5 with glycine.

17. Process according to any of Claims 9 to 15, characterized in that the taxane isolated is 10-deacetylbaccatin III and the eluent is a 2:1/dioxane water mixture buffered to pH 2.5 with glycine/HCI.

18. Process for the preparation of antitaxane polyclonal antibodies from egg yolk that comprises:
a) immunization of hens with a succinyl-taxane-albumin conjugate;
b) isolation of said antibodies from the egg yolk of the said hens.

19. Process according to Claim 18, characterized in that the said succinyl-taxane is succinyltaxol and the said albumin is bovine serum albumin.

20. Process according to Claim 18, characterized in that the said succinyl-taxane is succinyl-10-deacetylbaccatin III and the said albumin is bovine serum albumin.

21. Antitaxane polyclonal antibody obtainable by the process according to Claims 18 to 20.

22. Use of the antibody according to Claim 21 to isolate and determine taxanes in mixtures and biological fluids.

23. Use according to Claim 22, characterized in that the said determination is conducted by means of immunoaffinity chromatography or by means of competitive inhibition enzyme immunoassay (CIEIA).

24. Method for the determination of taxanes in samples of biological fluids or culture media, characterized in that the said sample is treated with a polyclonal antibody according to Claim 21 to give a taxane-antibody complex and the said taxane is then determined from the said complex.

25. Method according to Claim 24, characterized in that the said biological fluid is blood.

26. Method according to Claim 24 or Claim 25, characterized in that the determination of the said taxane is effected by immunoaffinity chromatography in which the said antibody is bound to the chromatographic support.

27. Method according to Claims 24 or Claim 25, characterized in that the determination of the said taxane is effected by competitive inhibition enzyme immunoassay (CIEIA).

28. Method according to any of Claims 24 to 27, characterized in that the taxane is taxol or 10-deacetylbaccatin III.

## Patentansprüche

1. Antikörper der avianen Vogel-IgY-Klasse, der Antikörperspezifität für ein das Taxan-Ringgerüst umfassendes Antigen aufweist.

2. Antikörper nach Anspruch 1, wobei das Antigen eine der folgenden Strukturformeln aufweist

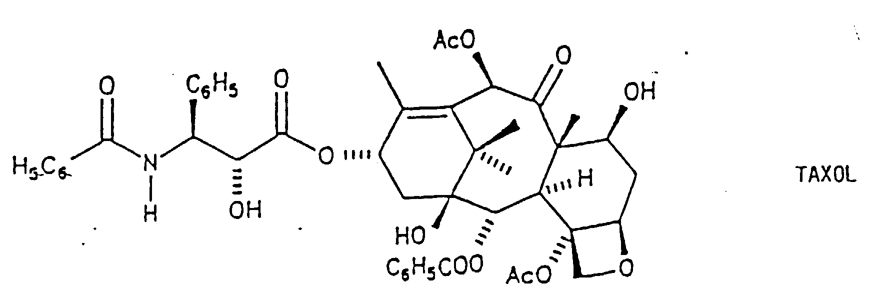

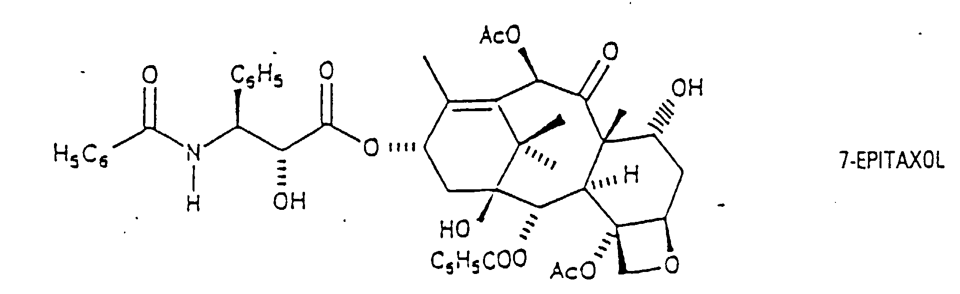

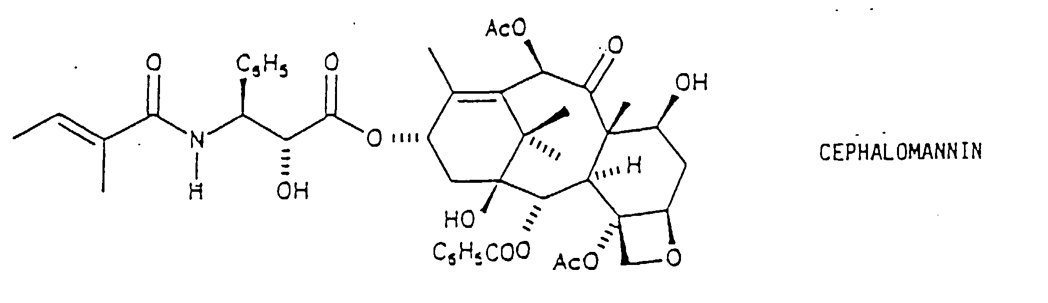

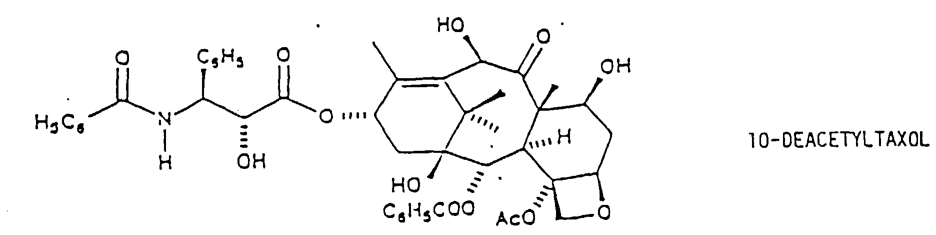

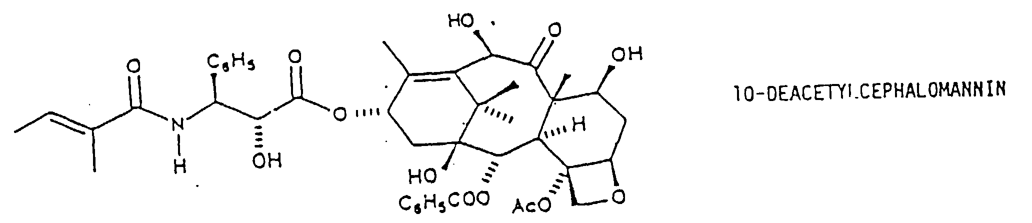

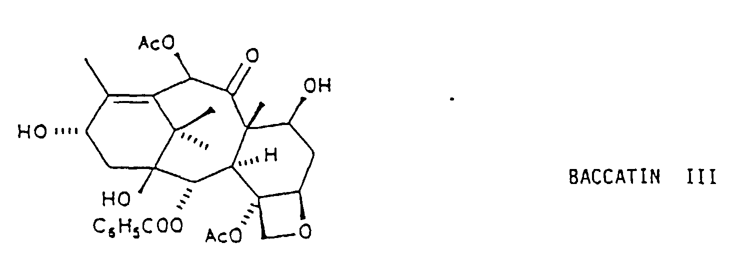

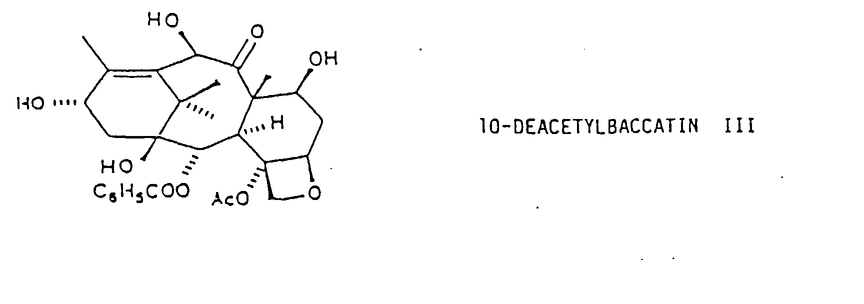

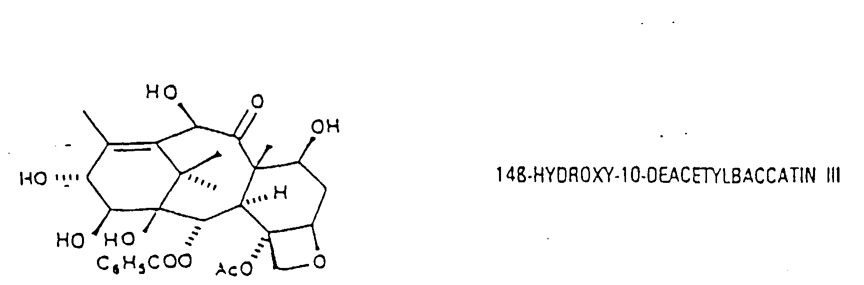

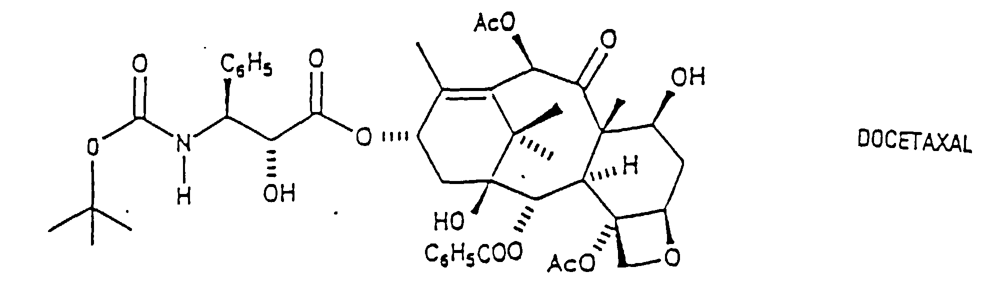

3. Antikörper nach Anspruch 1 oder 2, wobei es sich beim Antigen um Taxol oder um einen Taxol-Vorläufer handelt.

4. Antikörper nach Anspruch 3, wobei es sich beim Antigen um 10-Desacetylbaccatin III handelt.

5. Antikörper nach einem der vorstehenden Ansprüche, wobei es sich um Haushuhn-IgY handelt.

6. Verfahren zur Herstellung eines Antikörpers gemäß der Definition in einem der vorstehenden Ansprüche, umfassend das Reizen eines weiblichen Vogels mit dem Antigen und das Isolieren des IgY-Antikörpers aus den anschließend gelegten Eiern.

7. Verwendung eines Antikörpers nach einem der Ansprüche 1 bis 5 als analytisches Reagenz zum Nachweis und/oder zur Bestimmung einer Verbindung, die das Taxan-Ringgerüst umfaßt.

8. Verwendung eines Antikörpers nach einem der Ansprüche 1 bis 5 als präparatives Reagenz zum Einsatz zur Verwendung beim Isolieren oder Reinigen einer Verbindung, die das Taxan-Ringgerüst umfaßt.

9. Verfahren zur Reinigung eines Taxans, umfassend das Behandeln eines Gemisches, das dieses Taxan enthält, mit einem polyklonalen Antikörper gegen dieses Taxan, das anschließende Isolieren des Taxan-Antikörper-Komplexes und das Gewinnen des Taxans, dadurch gekennzeichnet, daß der polyklonale Antikörper aus einer Vogelspezies, insbesondere aus Hühnern, erhalten worden ist.

10. Verfahren nach Anspruch 9, dadurch gekennzeichnet, daß der polyklonale Antikörper an einem chromatographischen Träger für die Immunoaffinitätschromatographie fixiert ist.

11. Verfahren nach Anspruch 9, dadurch gekennzeichnet, daß es sich beim chromatographischen Träger um ein Harz mit einer mit Hydrazingruppen derivatisierten Methacryl-Struktur handelt.

12. Verfahren nach Anspruch 11, dadurch gekennzeichnet, daß die Elution des Trägers mit einem wäßrigen Gemisch von Dioxan oder einem aliphatischen Alkohol, das auf den pH-Wert 2,5 gepuffert ist, durchgeführt wird.

13. Verfahren nach Anspruch 12, dadurch gekennzeichnet, daß es sich beim Elutionsmittel um ein mit Glycin/HCl gepuffertes Gemisch aus Dioxan und Wasser handelt.

14. Verfahren nach Anspruch 9, dadurch gekennzeichnet, daß es sich bei dem Gemisch um einen Extrakt handelt, der aus einer Spezies der Gattung Taxus, ausgewählt unter, *Taxus baccata*, *T. brevifolia, T. wallichiana, T. hicksi, T. canadensis, T. hiamanensis* und *T. piramidalis*, erhalten worden ist.

15. Verfahren nach einem der Ansprüche 9 bis 14, dadurch gekennzeichnet, daß das isolierte Taxan aus der Gruppe Taxol, 10-Desacetylbaccatin III, 14-Hydroxy-10-desacetylbaccatin III, Baccatin III, Cephalomannin, 19-Hydroxy-10-desacetylbaccatin III und 7-Epitaxol ausgewählt ist.

16. Verfahren nach einem der Ansprüche 9 bis 14, dadurch gekennzeichnet, daß es sich beim isolierten Taxan um Taxol handelt und es sich beim Elutionsmittel um ein 1:1-Gemisch aus Dioxan und Wasser, das mit Glycin auf den pH-Wert 2,5 gepuffert ist, handelt.

17. Verfahren nach einem der Ansprüche 9 bis 15, dadurch gekennzeichnet, daß es sich bei dem isolierten Taxan um 10-Desacetylbaccatin III handelt und es sich beim Elutionsmittel um ein 2:1-Gemisch aus Dioxan/Wasser, das mit Glycin/HCl auf den pH-Wert 2,5 gepuffert ist, handelt.

18. Verfahren zur Herstellung von polyklonalen Antitaxan-Antikörpern aus Eidotter, umfassend:
a) das Immunisieren von Hühnern mit einem Succinyltaxan-Albumin-Konjugat; und
b) das Isolieren der Antikörper aus dem Eidotter der Hühner.

19. Verfahren nach Anspruch 18, dadurch gekennzeichnet, daß es sich beim Succinyltaxan um Succinyltaxol und beim Albumin um Rinderserumalbumin handelt.

20. Verfahren nach Anspruch 18, dadurch gekennzeichnet, daß es sich beim Succinyltaxan um Succinyl-10-desacetylbaccatin III und beim Albumin um Rinderserumalbumin handelt.

21. Polyklonaler Antitaxan-Antikörper, erhältlich nach dem Verfahren gemäß den Ansprüchen 18 bis 20.

22. Verwendung des Antikörpers nach Anspruch 21 zum Isolieren und zum Bestimmen von Taxanen in Gemischen und biologischen Flüssigkeiten.

23. Verwendung nach Anspruch 22, dadurch gekennzeichnet, daß die Bestimmung durch Immunoaffinitätschromatographie oder durch einen kompetitiven Hemm-Enzymimmunoassay (CIEIA) durchgeführt wird.

24. Verfahren zur Bestimmung von Taxanen in Proben von biologischen Flüssigkeiten oder Kulturmedien, dadurch gekennzeichnet, daß die Probe mit einem polyklonalen Antikörper gemäß Anspruch 21 behandelt wird, wodurch man einen Taxan-Antikörper-Komplex erhält, und das Taxan anschließend im Komplex bestimmt wird.

25. Verfahren nach Anspruch 24, dadurch gekennzeichnet, daß es sich bei der biologischen Flüssigkeit um Blut handelt.

26. Verfahren nach Anspruch 24 oder 25, dadurch gekennzeichnet, daß die Bestimmung des Taxans durch Immunoaffinitätschromatographie durchgeführt wird, bei der der Antikörper an den chromatographischen Träger gebunden wird.

27. Verfahren nach Anspruch 24 oder 25, dadurch gekennzeichnet, daß die Bestimmung des Taxans durch einen kompetitiven Hemm-Enzymimmunoassay (CIEIA) durchgeführt wird.

28. Verfahren nach einem der Ansprüche 24 bis 27, dadurch gekennzeichnet, daß es sich beim Taxan um Taxol oder 10-Desacetylbaccatin III handelt.

## Revendications

1. Anticorps de la classe des IgY aviaires, ayant une spécificité d'anticorps pour un antigène qui comprend le squelette cyclique du taxane.

2. Anticorps selon la revendication 1, pour lequel l'antigène a l'une des formules développées suivantes:

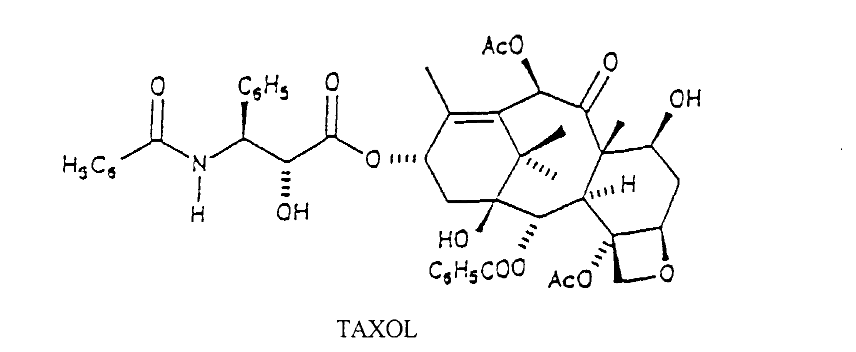

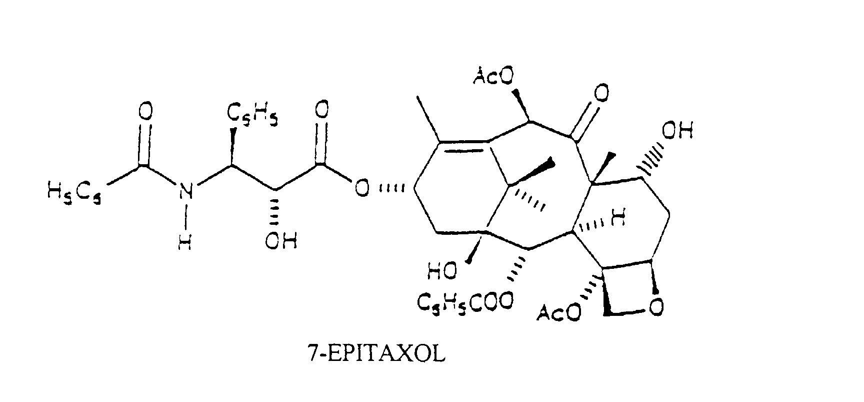

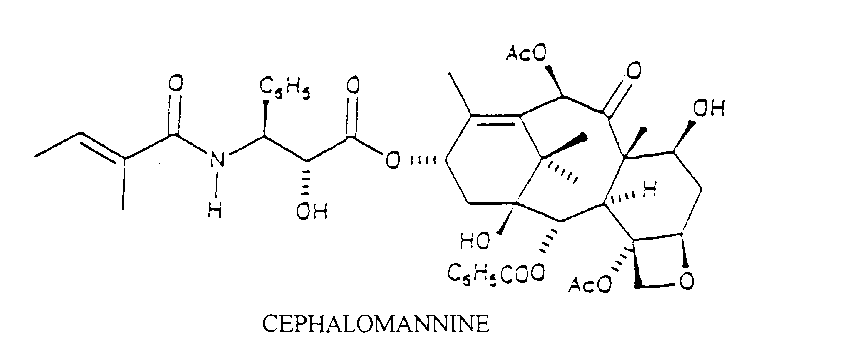

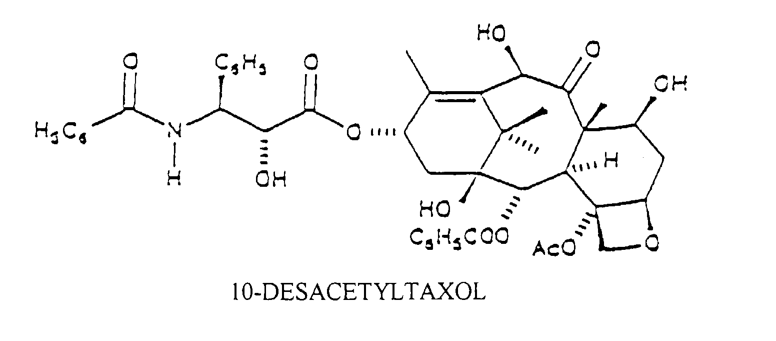

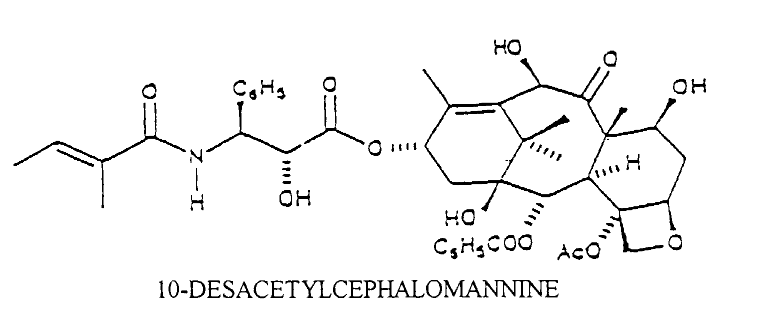

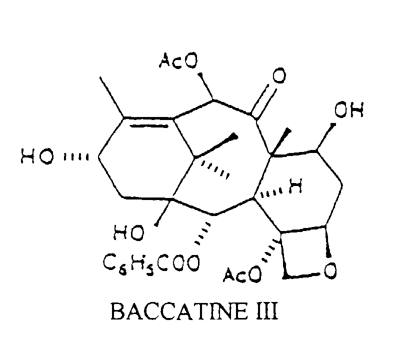

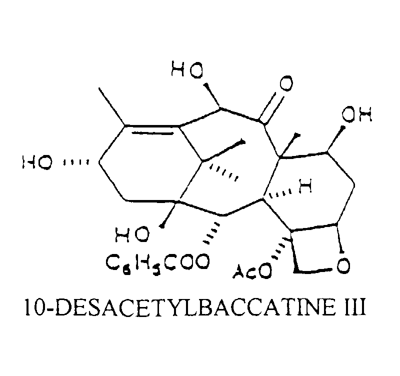

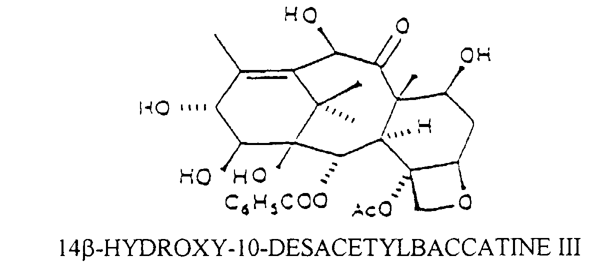

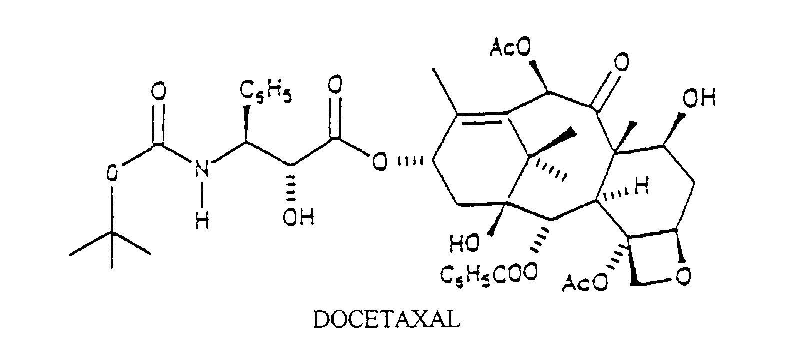

3. Anticorps selon la revendication 1 ou la revendication 2, pour lequel l'antigène est le taxol ou un précurseur du taxol.

4. Anticorps selon la revendication 3, pour lequel l'antigène est la 10-désacétylbaccatine III.

5. Anticorps selon l'une quelconque des revendications précédentes, qui est une IgY de poule domestique.

6. Procédé de production d'un anticorps tel que défini dans l'une quelconque des revendications précédentes, qui comprend la provocation d'une femelle aviaire par ledit antigène et l'isolement de l'anticorps IgY à partir des oeufs pondus par la suite.

7. Utilisation d'un anticorps selon l'une quelconque des revendications 1 à 5 comme réactif d'analyse pour la détection et/ou le dosage d'un composé comprenant le squelette cyclique du taxane.

8. Utilisation d'un anticorps selon l'une quelconque des revendications 1 à 5 comme réactif de préparation à utiliser dans l'isolement ou la purification d'un composé comprenant le squelette cyclique du taxane.

9. Procédé de purification d'un taxane, comprenant le traitement d'un mélange contenant ledit taxane avec un anticorps polyclonal dirigé contre ledit taxane, suivi de l'isolement du complexe taxane-anticorps et de la récupération du taxane, caractérisé en ce que ledit anticorps polyclonal est obtenu à partir d'une espèce aviaire, en particulier de la poule.

10. Procédé selon la revendication 9, caractérisé en ce que ledit anticorps polyclonal est fixé sur un support chromatographique pour chromatographie d'immunoaffinité.

11. Procédé selon la revendication 9, caractérisé en ce que ledit support chromatographique est une résine à structure méthacrylique modifiée avec des groupes hydrazine.

12. Procédé selon la revendication 11, caractérisé en ce que l'élution dudit support s'effectue avec un mélange aqueux de dioxane ou d'un alcool aliphatique tamponné à pH 2,5.

13. Procédé selon la revendication 12, caractérisé en ce que l'éluant est un mélange de dioxane et d'eau tamponné avec de la glycine/HCl.

14. Procédé selon la revendication 9, caractérisé en ce que ledit mélange est un extrait obtenu à partir d'une espèce du genre Taxus choisie dans le groupe constitué par *Taxus baccata, T. brevifolia, T*. *wallichiana, T. hicksi, T. canadensis, T*. *hiamanensis, T. piramidalis.*

15. Procédé selon l'une quelconque des revendications 9 à 14, caractérisé en ce que le taxane isolé est choisi dans le groupe constitué par le taxol, la 10-désacétylbaccatine III, la 14-hydroxy-10-désacétylbaccatine III, la baccatine III, la céphalomannine, la 19-hydroxy-10-désacétylbaccatine III et le 7-épitaxol.

16. Procédé selon l'une quelconque des revendications 9 à 14, caractérisé en ce que le taxane isolé est le taxol et en ce que l'éluant est un mélange 1:1 de dioxane et d'eau tamponné à pH 2,5 avec de la glycine.

17. Procédé selon l'une quelconque des revendications 9 à 15, caractérisé en ce que le taxane isolé est la 10-désacétylbaccatine III et en ce que l'éluant est un mélange 2:1 de dioxane et d'eau tamponné à pH 2,5 avec de la glycine/HCl.

18. Procédé de préparation d'anticorps polyclonaux antitaxane à partir de jaune d'oeuf, qui comprend:
a) l'immunisation de poules avec un conjugué succinyltaxane-albumine;
b) l'isolement desdits anticorps à partir du jaune d'oeuf desdites poules.

19. Procédé selon la revendication 18, caractérisé en ce que ledit succinyltaxane est un succinyltaxol et en ce que ladite albumine est la sérum-albumine bovine.

20. Procédé selon la revendication 18, caractérisé en ce que ledit succinyltaxane est une succinyl-10-désacétylbaccatine III et en ce que ladite albumine est la sérum-albumine bovine.

21. Anticorps polyclonal antitaxane pouvant être obtenu par le procédé selon les revendications 18 à 20.

22. Utilisation de l'anticorps selon la revendication 21 pour l'isolement et la détermination de taxanes dans des mélanges et des liquides biologiques.

23. Utilisation selon la revendication 22, caractérisée en ce que ladite détermination s'effectue à l'aide d'une chromatographie d'immunoaffinité ou à l'aide d'une analyse immuno-enzymatique faisant intervenir une inhibition compétitive (CIEIA).

24. Procédé de détermination de taxanes dans des échantillons de liquides biologiques ou de milieux de culture, caractérisé en ce que l'on traite ledit échantillon avec un anticorps polyclonal selon la revendication 21 pour obtenir un complexe taxane-anticorps et on détermine ensuite ledit taxane à partir dudit complexe.

25. Procédé selon la revendication 24, caractérisé en ce que ledit liquide biologique est le sang.

26. Procédé selon la revendication 24 ou la revendication 25, caractérisé en ce que la détermination dudit taxane s'effectue par une chromatographie d'immunoaffinité dans laquelle ledit anticorps est lié au support chromatographique.

27. Procédé selon la revendication 24 ou la revendication 25, caractérisé en ce que la détermination dudit taxane s'effectue par analyse immuno-enzymatique faisant intervenir une inhibition compétitive (CIEIA).

28. Procédé selon l'une quelconque des revendications 24 à 27, caractérisé en ce que le taxane est le taxol ou la 10-désacétylbaccatine III.
